# EUROPEAN PATENT SPECIFICATION

(11) **EP 4 627 807 B1**
(45) Date of publication and mention of the grant of the patent: **24.06.2026**
(21) Application number: 24712130.4
(22) Date of filing: 07.02.2024
(51) Int. Cl.: H04R 25/00

(54) **INTRAOSSEOUS HEARING IMPLANT**
INTRAOSSALES HÖRIMPLANTAT
IMPLANT AUDITIF INTRA-OSSEUX

(30) Priority: 08.02.2023 PL 44371023
(43) Date of publication of application: 08.10.2025
(73) Proprietor: Politechnika Warszawska (WUT), 00 661 Warszawa (PL)
(72) Inventor: BORKOWSKI, Pawel, Warszawa (PL)
(74) Representative: Rybarczyk, Dariusz Pawel
(86) International application number: PCT/IB2024/051132
(87) International publication number: WO 2024/166013

(56) References cited:
- WO-A2-2010/133706
- US-A- 5 702 342
- US-B1- 6 540 661

## Description

The subject-matter of the invention is an intraosseous hearing implant, which is part of an auditory perception stimulation system using bone conduction or part of a bone conduction - based hearing aid, as used in auditory prosthetics.

Hearing implants are part of hearing aids using the phenomenon of bone conduction of sound. Competitive bone conduction implants have been known and implanted in patients for many years. The bone conduction hearing implants that are used today operate by exciting vibrations on the outer surface of the skull, and these then excite structures in the inner ear.

Bonebridge BCI 602 hearing implant, MED-EL, is known from publications, the implant being placed under the skin on the surface of the skull, capturing sounds from the environment and transmitting them to the inner ear via the skull bone, bypassing the damaged outer and middle ear. For people with single-sided deafness to collect sound vibrations on the deaf side and transfer them to the inner ear on the hearing side.

BAHA (Bone Anchored Hearing Aid) system is known from publications, the system being based on bone conduction and using titanium implants fixed to the temporal bones, which act as a link between the sound processor - which converts sound waves into vibrations - and the skull bones, with the latter transferring said vibrations forward to the inner ear [publication: Wróbel M., Szyfter W., "BAHA^{®} system in hearing impairment fitting." Post 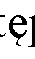 py w chirurgii glowy i szyi/Advances in Head and Neck Surgery, 2009, vol. 8, no. 1, pp. 1-9],

A hearing aid as disclosed in patent PL237901 Bl is known from the prior art, the hearing aid for stimulation of the inner ear by means of bone conduction. The hearing aid has a speech processor connected directly or via a connecting element with an intraosseous implant. In this hearing aid, the implant, which is placed in the bone mass inside the curvature of the superior semicircular canal of the labyrinth, is in the shape of an elongated cone (1). On the narrower side, the implant is terminated with a threaded shank (2) which is 4 - 5 mm long and has a diameter of 0.6 1.0 mm, while on the wider side, the implant has a stabilising part (3) provided with projections that enable the implant to be screwed in, and a platform (5) above the stabilising part (3), the platform connecting the implant to the housing (6) of the speech processor equipped with a source.

An intraosseous hearing implant (PL71904 Y1 ) is known from the prior art, the implant being part of a bone conduction-based auditory perception stimulation system or part of a bone conduction-based hearing aid. The implant is in the shape of an elongated cone, consisting of a base part (1) which, at the thinner end, has a shank (2) with a metric fine thread. On the other, wider end of the base part (1), there is a tetrahedral positioning element (3), with a spacer (4) seated thereon, the spacer supporting a platform (5) having a diameter slightly longer than the diameter of the spacer (4) and provided in its central part with a blind, threaded hole enabling the speech processor housing to be screwed into the platform.

An intraosseous hearing implant (PL71905 Y1) is known from the prior art, the implant being part of a bone conduction-based auditory perception stimulation system or part of a bone conduction-based hearing aid. The implant is in the shape of an elongated cone, consisting of a base part (1) which, at the thinner end, has a shank (2) on which there is a metric fine thread. On the other, wider end, the base part (1) has a hexahedral positioning element (3), with a spacer (4) seated thereon, the spacer supporting a platform (5) having a diameter slightly longer than the spacer and having a magnetic layer (6) thereon enabling the implant to be connected to the speech processor housing.

Other solutions similar in terms of the location where the hearing implant is implanted are also known in the prior art, for example: PL71906 Y1, PL71907 Y1, PL71908 Y1, PL71909 Y1.

Publications are known about the use of electromechanical exciters to stimulate the round window or middle ear ossicles; [Shin D.H., Kim J.H., Gottlieb P., Vaisbuch Y., Puria S., Cho J., Seong K.W., Comparative study of efficiency and characteristics of FMT and DRT installed in human cadavers for round-window stimulation, Scientific Reports, vol. 11: 16775, 2021; Shin D.H., Ki Woong S., Puria S., Kyu-Yup L., Cho J., A tri-coil bellows-type round window transducer with improved frequency characteristics for middle-ear implants, Hearing Research, vol. 341, 2016, pp. 144-154; Stieger C., Bernhard H., Waeckerlin D., Kompis M., Burger J., Haeusler R., Human temporal bones versus mechanical model to evaluate three middle ear transducers, J Rehabil. Res. Dev., 2007; vol. 44, no. 3, pp. 407-15; Min-Kyu K., I1- Yong P., Byung-Scop S., Jin-Ho Ch., Fabrication and optimal design of differential electromagnetic transducer for implantable middle ear hearing device, Biosensors and Bioelectronics, 2006, vol. 21, no. 11, pp. 2170-2175],

Publications on bone conduction stimulation direction testing solutions are known, such as: [Borkowski P., Marek P., Niemczyk K., Lachowska M., Kwacz M., Wysocki J., Bone conduction stimulation of the otic capsule: a finite element model of the temporal bone, Acta of Bioengineering and Biomechanics, vol. 21, no. 3, 2019, pp. 75-86; Zhao M., Fridberger A., Stenfelt S., Vibration direction sensitivity of the cochlea with bone conduction stimulation in guinea pigs, Scientific Reports, 2021, 11: 2855, doi: 10.1038/s41598-021-82268-3],

Websites: https://revbio.com/publications/; https://revbio.com/tetranite-technology/; https://revbio.com/publications/, as accessed on 03 January 2023, disclose the types of adhesive to be used in fixing the implant to the bone.

WO 2010133706 A2 discloses a hearing instrument for round or oval window stimulation, comprising an audio signal source, an audio signal processing unit for processing the audio signals provided by the audio signal source, and implantable stimulation assembly for stimulating a round window or an oval window of a patient according to the processed audio signals. The stimulation assembly comprises an electromechanical actuator for vibrating an output member according to the processed audio signals, a support member to be fixed at the patient's skull, and a lever element having a proximal end portion, a distal end portion and an intermediate portion connecting the proximal end portion and the distal end portion. The actuator is fixed at the support member, wherein the intermediate portion is supported by the support member in manner enabling a pivoting motion of the lever element. The output member is arranged for imparting an pivoting motion to the lever element by acting on the proximal portion. The competitive bone conduction implants that are used today operate by exciting vibrations on the outer surface of the skull, and these then excite structures in the inner ear. In the solution according to the invention, vibrations are excited almost directly in the inner ear. The solution according to the invention uses two joints, enabling the direction of vibration forcing to be adjusted and facilitating implantation. Characteristic of the developed design solution is the fact that it is possible to set a specific direction of stimulation, favourable in terms of the intensity of vibration excitation in the cochlea of the inner ear. Placing the exciter close to the inner ear is preferred because it has the effect of the implant being miniaturised due to a reduction in the force required for stimulation via bone conduction, resulting in lower vibration energy. The miniaturisation of the implant thus has the effect of extending the life of the power supply battery when using the hearing aid on a daily basis. The optional use of implants on both sides of the head, placed close to the inner ear and thus not causing vibration of the entire skull, can have a positive effect on understanding speech in difficult acoustic conditions and improve the localisation of the sound source. Placing the implant completely under the skin reduces the risk of complications related to inflammation at the implant site. Furthermore, the socket that fixes the implant to the bone is attached using an adhesive binder, which eliminates the need for additional invasive procedures in the labyrinth capsule.

The invention is defined by claim 1. The essence of the inventive intraosseous hearing implant, which is part of a bone conduction-based system for the stimulation of auditory perception, is that the intraosseous hearing implant consists of a mechanical vibration exciter, the exciter comprising an electromechanical transducer and comprising a shank and, at the opposite end, a spherical tip coated with a polymer shield, with a tightening nut fixed on the tip towards the bone of the skull, and further, a fixing socket which is operationally tangential to the tightening nut, and whose surface tangential to the bone of the skull is coated with a layer of bioactive coating and whose base is flush with the spherical tip of the shank of the mechanical vibration exciter, and which is attached to the bone of the skull by means of an adhesive binder being a biomaterial, and *the essence is also that* the shank of the mechanical vibration exciter is connected by a sliding ball comprising a through-hole, through which the shank of the mechanical vibration exciter passes, where the shank of the exciter is connected to an adjusting pin seated in the socket of a fixing plate, through a stationary holder and a rotary holder both placed on the top of the adjusting pin, in which the sliding ball is placed.

Preferably, the fixing socket has a cylindrical shape whose base surface tangential to the surface of the bone of the skull is flat.

Preferably, the fixing socket has a threaded outer cylindrical surface. Preferably, the fixing socket is made of titanium.

Preferably, the adhesive binder is an adhesive, osteoconductive and bioresorbable biomaterial. Preferably, the base surface of the fixing socket tangential to the surface of the bone of the skull is coated with a layer of hydroxyapatite.

Preferably, the shank of the mechanical vibration exciter has a cylindrical shape.

Preferably, the shank of the mechanical vibration exciter is made of titanium.

Preferably, the shield of the spherical tip of the shank of the mechanical vibration exciter is made of Teflon.

Preferably, the electromechanical transducer chamber has a shape of a cylinder having a diameter d_{w} = 5 mm and a height 1_{w} = 6 mm.

Preferably, the tightening nut has a cylindrical shape on the interface with the fixing socket. Preferably, the tightening nut has a spherical recess in the region of contact with the spherical tip of the shank.

Preferably, the adjusting pin has a cylindrical shape.

Preferably, the adjusting pin has a threaded outer surface on the surface in contact with the fixing plate.

Preferably, the adjusting pin, on the side of the shank of the mechanical vibration exciter, has a stationary holder and a rotary holder with a spherical inner surface.

Preferably, the rotary holder is rotatable with up to 360° of rotary motion.

Preferably, the adjusting pin with the stationary holder is made of titanium alloy.

Preferably, the adjusting pin with the rotary holder is made of UHMWPE polyethylene. Preferably, the adjusting pin has a hole together with a spherical groove, and a shank of the holder, and a spherical tip of the shank of the holder.

Preferably, the fixing plate, which is approximately Y -shaped, has curved edges and a central surface between the first arm and the second arm and the third arm.

Preferably, the axis of the third arm is at an angle of a = 120° with respect to the axis of the first arm and the axis of the second arm.

Preferably, the fixing plate on the central surface in its central part comprises a cylindrical socket threaded internally over the entire surface.

Preferably, the first arm and the second arm and the third arm of the fixing plate comprises working holes.

Preferably, the fixing plate is made of titanium alloy.

The intraosseous hearing implant according to the invention is shown in the embodiment and in the drawing in which:
Fig. 1 shows a hearing implant with marked components in a perspective view; Fig. 2a shows an embodiment of a hearing implant in cross-sectional view through the plane containing the axes of the shank of the exciter and the adjusting pin together with the stationary holder;
Fig. 2b shows another embodiment of a hearing implant in cross-sectional view through the plane comprising the axes of the shank of the exciter and the adjusting pin together with the rotary holder;
Fig. 3 shows a hearing implant located in a hole in the right temporal bone.

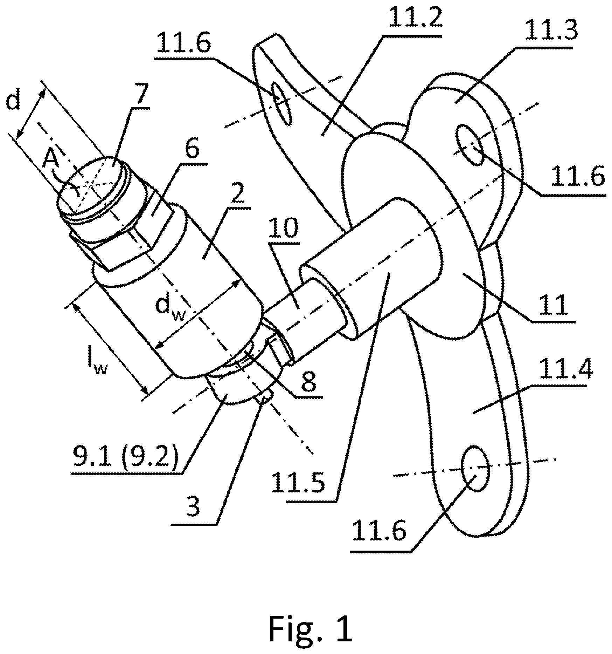

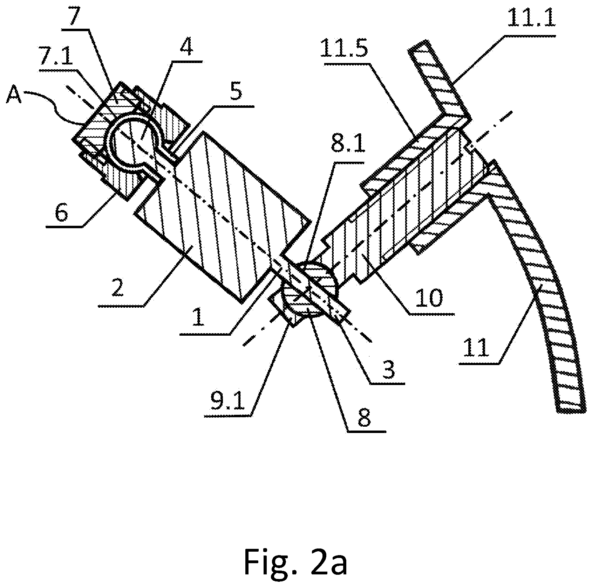

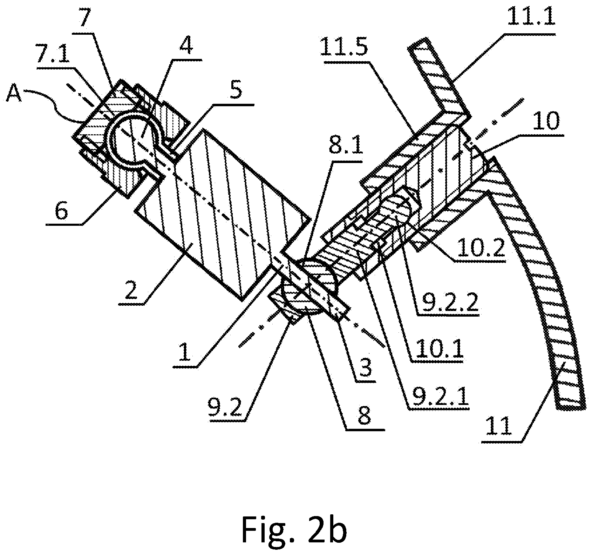

The intraosseous hearing implant (Fig. 1, 2a and 2b) consists of a mechanical vibration exciter 1, the exciter comprising an electromechanical transducer 2 and comprising a shank 3 and, at the opposite end, a spherical tip 4 coated with a shield 5, with a tightening nut 6 fixed on the tip, and further, a fixing socket 7 which is operationally tangential to the tightening nut 6, and whose surface tangential to the bone of the skull CZ is coated with a layer of bioactive coating, and whose base 7.1 is flush with the shield 5 of the spherical tip 4 of the shank 3 of the mechanical vibration exciter 1. The fixing socket 7 is attached to the bone of the skull CZ by means of an adhesive binder. A sliding ball 8 comprising a through hole 8.1, located in a stationary holder 9. 1 or a rotary holder 9.2 of the adjusting pin 10, is located on the shank 3 of the mechanical vibration exciter 1. The adjusting pin 10 is seated in the socket 11.5 of the fixing plate 11.

The fixing socket 7, made of titanium, has a cylindrical shape and its base (surface A, Fig. 1) is coated with a layer of bioactive hydroxyapatite coating enabling bone growth and is flat and tangential to the surface of the skull CZ. The diameter of the base of the fixing socket 7 is d = 3.4 mm. The fixing socket 7 comprises a threaded outer cylindrical surface. The fixing socket 7 is attached to the bone by means of an adhesive binder, e.g. self-binding Tetranite biomaterial (RevBio Inc., USA), with adhesive, osteoconductive and bioresorbable characteristics, for use in a moist surgical environment. The fixing socket 7 ensures a stable connection between the hearing implant and the bone.

The tightening nut 6 of titanium alloy (other embodiments use cobalt-chromium alloy or noble alloys based on gold or platinum) has a cylindrical shape in the interface with the fixing socket 7 and its outer edges in cross-section form an isosceles hexagon, with the tightening nut 6 having a spherical recess and a threaded inner cylindrical surface. The tightening nut 6 is intended to eliminate play between the spherical tip 4 of the shank 3 of the mechanical vibration exciter 1 and the fixing socket 7, while still permitting rotational movement.

The mechanical vibration exciter 1 placed under the external surface of the bone of the skull close to the inner ear, made of titanium alloy (other embodiments use a cobalt-chromium alloy or noble alloys based on gold or platinum), has a cylindrical shape and the shield 5 of the spherical tip 4 of the exciter 1 is made of Teflon (in another embodiment it is made of UHMWP polyethylene), with the radius of the spherical tip 4 including the shield 5 being smaller by about 0.01-0.5 mm than the radius of the recess in the fixing socket 7 and the tightening nut 6. The base surface A of fixing socket 7 on the side of the bone is about 9 mm2 . The shank 3 of the mechanical vibration exciter 1 transmits vibrations from the electromechanical transducer 2 to the bone and maintains the set direction of stimulation. The purpose of the elastomeric shield 5 of the spherical tip 4 of the shank 3 is to ensure, due to the low coefficient of friction, the correct kinematics of the joint and to eliminate any run-out caused by undesired separation of the fixing socket 7 and the tip of the shank 4 due to vibration of the mechanical vibration exciter 1, with the hearing implant still being able to transmit force to the inner ear.

The electromechanical transducer 2 is fixed on the shank 3 of the exciter 1 and has an oscillating mass and a set of electromagnets (not shown). The electromechanical transducer 5 chamber has a shape of a cylinder having a diameter d_{w} = 5 mm and a length 1_{w} = 6 mm. The electromechanical transducer 2 converts the electrical signal into mechanical vibrations of the shank 3 of the mechanical vibration exciter 1 in the longitudinal direction.

A sliding ball 8 made of Teflon or in another embodiment of a biocompatible low-friction polymer, e.g. UHMWPE polyethylene, is situated in a stationary holder 9.1 placed on the top of the adjusting pin 10 (Fig. 2a), wherein the sliding ball 8 is rotatable relative to the holder 9.1.

In another embodiment (Fig. 2b), the sliding ball 8 is situated in the rotary holder 9.2. The sliding ball 8 has a cylindrical hole 8.1 for the shank 3 of the exciter 1 (Fig. 2a, Fig. 2b). The task of the sliding ball 8 is to ensure free travel of the shank 3 in the hole 8. 1 of the sliding ball 8 and to enable the angle between the axes of the adjusting pin 10 and the shank 3 of the mechanical vibration exciter 1 to be changed.

The adjusting pin 10 made of titanium alloy (for example, as disclosed in Fig. 2a) or UHMWPE polyethylene (for example, as disclosed in Fig. 2b) has a cylindrical shape and a threaded outer surface in the part in contact with the fixing plate 11 (Figs. 2a, 2b). On the side of the shank 3 of the mechanical vibration exciter 1, the adjusting pin 10 comprises a non- rotary holder 9.1 (Fig. 2a) or a rotary holder 9.2 (Fig. 2b). The rotary holder 9.2 capable of rotating up to 360° comprises a shank 9.2.1 terminating opposite to the holder 9.2 with a spherical tip 9.2.2, the shank 9.2.1 together with the spherical tip 9.2.2 being placed in a cylindrical hole 10.1 of the adjusting pin 10, towards the base of which there is a spherical groove 10.2 for seating the spherical tip 9.2.2 of the shank 9.2. 1 of the rotary holder 9.2 therein to prevent axial displacement of the spherical tip 9.2.2 during rotation of the spherical tip 9.2.2 about the axis of the adjusting pin 10. The diameters of the hole 10.1 and the shank 9.2.1 are graduated to ensure that the two parts slip over each other when rotating and to enable the spherical tip 9.2.2 to be pressed into the spherical groove 10.2. The adjusting pin 10 makes it possible to modify the direction of the force transmitted to the inner ear by the mechanical vibration exciter 1.

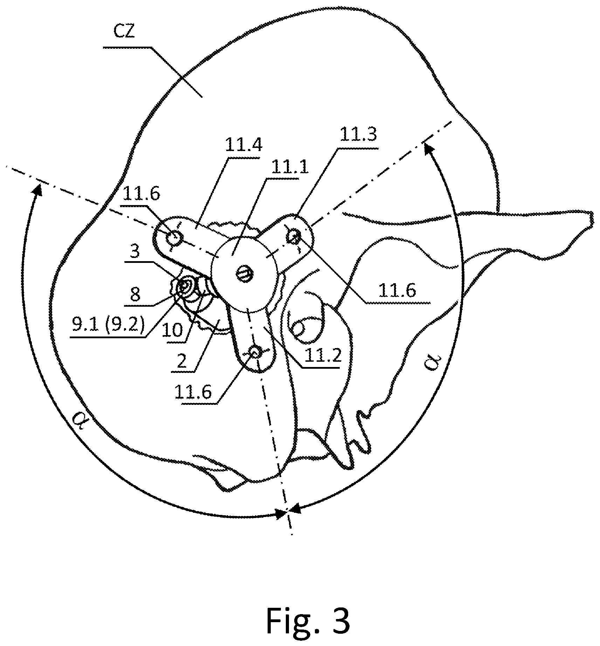

The fixing plate 11 of titanium alloy (or, in another embodiment, of cobalt-chromium alloy, or optionally of noble alloys based on gold or platinum), having approximately a Y -shape, has curved edges and a central surface 11.1 between the arms: the first arm 11.2, the second arm 11.3 and the third arm 11.4. The axis of the first arm 11.2 is at an angle of a = 120° to the axis of the second arm 11.3 and of the third arm 11.4. On the central surface 11.1, there is an internally threaded cylindrical socket 11.5 for the insertion of the adjusting pin 10 (Figs. 2a, 2b). On the surface of all arms 11.2, 11.3, 11.4, there are three working holes 11.6 for the screws fixing the hearing implant to the external surface of the skull CZ, arranged at the intersection of the circle of 22 mm diameter with the axes of the arms 11.2, 11.3 and 11.4. The central surface 11.1 has the shape of a circular plate with a diameter of 10 mm and a thickness of 1 mm. The position of the arms 11.2, 11.3 and 11.4 is anatomically adapted to the bone structure of the skull CZ. The first 11.2 and second 11.3 arms are on an arch between the edge of the zygomatic bone and the mastoid process (Fig. 3). On the central surface 11.1 of the fixing plate 11, there is a receiving coil and a magnet (not shown). The task of the fixing plate 11 is to fix the adjusting pin 10 and protect the remaining parts of the hearing implant from unwanted collapse into the skull CZ following trauma.

Electrical energy to the electromechanical transducer 2 (Fig. 1) is transmitted without contact via a system of magnetically coupled coils, as in the Bonebridge implant. The sound processor is placed on the skin of the skull (not shown) and comprises a microphone, battery, magnet and primary coil. The sound processor is fixed on the skin by means of a magnet placed on the central surface 11.1 of the fixing plate 11 (Fig. 3), where there is also a secondary coil, connected by wires (not shown) to the electromechanical transducer 2.

During the operation to implant the intraosseous hearing implant, a hole is made in the temporal bone on the external surface of the skull CZ, the fixing socket 7 is adhered to the bone close to the inner ear using an adhesive binder, the shank of the mechanical vibration exciter 1 is fixed by tightening the tightening nut 6, the fixing plate 11 is adjusted, the adjusting pin 10 is regulated, the shank 3 is guided through the hole 8.1 in the sliding ball 8 and the fixing plate 11 is screwed to the bone of the skull CZ.

In another embodiment, the method of mounting the intraosseous hearing implant involves adhering the pre-assembled shank 3 with the fixing socket 7 to the bone of the skull CZ, and then adjusting the fixing plate 11, regulating the adjusting pin 10, guiding the shank 3 through the hole 8. 1 in the sliding ball 8 and screwing the fixing plate 11 to the bone of the skull.

### List of numeric designations in the drawing:

- 1: Mechanical vibration exciter
- 2: Electromechanical transducer
- 3: Shank of the mechanical vibration exciter
- 4: Spherical tip of the shank
- 5: Shield of the spherical tip of the shank
- 6: Tightening nut
- 7: Fixing socket
- 7.1: Surface of the spherical recess of the fixing socket
- 8: Sliding ball
- 8.1: Hole of the sliding ball
- 9. 1: Stationary holder
- 9.2: Rotary holder
- 9.2.1: Shank of the holder
- 9.2.2: Spherical tip of the shank of the holder
- 10: Adjusting pin
- 10.1: Hole in the adjusting pin
- 10.2: Spherical groove in the hole of the adjusting pin
- 11: Fixing plate
- 11.1: Central surface
- 11.2: First arm of the fixing plate
- 11.3: Second arm of the fixing plate
- 11.4: Third arm of the fixing plate
- 11. 5: Socket of the fixing plate
- 11.6: Working holes of the fixing plate
- A: Surface of the fixing socket contacting the bone
- CZ: Skull

## Claims

1. An intraosseous hearing implant, which is part of a bone conduction-based system for the stimulation of auditory perception, and consists of a mechanical vibration exciter (1), the exciter comprising an electromechanical transducer (2) and comprising a shank (3) and, at the opposite end, a spherical tip (4), wherein the shank (3) of the mechanical vibration exciter (1) is connected by a sliding ball (8) comprising a through-hole (8.1), through which the shank (3) of the mechanical vibration exciter (1) passes, **characterised in that** the spherical tip (4) is coated with a polymer shield (5), with a tightening nut (6) fixed on the tip towards the bone of the skull (CZ), and further, a fixing socket (7) which is operationally tangential to the tightening nut (6), and whose surface tangential to the bone of the skull (CZ) is coated with a layer of bioactive coating and whose base is flush with the shield (5) of the spherical tip (4) of the shank (3) of the mechanical vibration exciter (1), and which is attached to the bone of the skull (CZ) by means of an adhesive binder being a biomaterial, wherein the shank (3) of the mechanical vibration exciter (1) is connected to an adjusting pin (10) seated in the socket (11.5) of a fixing plate (11), through a stationary holder (9.1) and a rotary holder (9.2) both placed on the top of the adjusting pin (10), in which the sliding ball (8) is placed.

2. The intraosseous hearing implant of claim 1, **characterised in that** the fixing socket (7) has a cylindrical shape, whose base surface (A) tangential to the surface of the bone of the skull (CZ) is flat.

3. The intraosseous hearing implant of claim 1, **characterised in that** the fixing socket (7) has a threaded outer cylindrical surface.

4. The intraosseous hearing implant of claim 1, **characterised in that** the fixing socket (7) is made of titanium.

5. The intraosseous hearing implant of claim 1, **characterised in that** the adhesive binder is an adhesive, osteoconductive and bioresorbable biomaterial.

6. The intraosseous hearing implant of claim 1, **characterised in that** the base surface (A) of the fixing socket (7) tangential to the surface of the bone of the skull (CZ) is coated with a layer of hydroxyapatite.

7. The intraosseous hearing implant of claim 1, **characterised in that** the shank (3) of the mechanical vibration exciter (1) has a cylindrical shape.

8. The intraosseous hearing implant of claim 1, **characterised in that** the shank (3) of the mechanical vibration exciter (1) is made of titanium.

9. The intraosseous hearing implant of claim 1, **characterised in that** the shield (5) of the spherical tip (4) of the shank (3) of the mechanical vibration exciter (1) is made of Teflon.

10. The intraosseous hearing implant of claim 1, **characterised in that** the electromechanical transducer (2) chamber has a shape of a cylinder having a diameter d_{w} = 5 mm and a height 1_{w} = 6 mm.

11. The intraosseous hearing implant of claim 1, **characterised in that** the tightening nut (6) has a cylindrical shape on the interface with the fixing socket.

12. The intraosseous hearing implant of claim 1, **characterised in that** the tightening nut (6) has a spherical recess (7.1) in the region of contact with the spherical tip (4) of the shank (3).

13. The intraosseous hearing implant of claim 1, **characterised in that** the adjusting pin (10) has a cylindrical shape.

14. The intraosseous hearing implant of claim 1, **characterised in that** the adjusting pin (10) has a threaded outer surface on the surface in contact with the fixing plate (11).

15. The intraosseous hearing implant of claim 1, **characterised in that** the adjusting pin (10), on the side of the shank (3) of the mechanical vibration exciter (1), has a stationary holder (9.1) and a rotary holder (9.2) with a spherical inner surface.

16. The intraosseous hearing implant of claim 1, **characterised in that** the rotary holder (9.2) is rotatable with up to 360° of rotary motion.

17. The intraosseous hearing implant of claim 1, **characterised in that** the adjusting pin (10) with the stationary holder (9. 1) is made of titanium alloy.

18. The intraosseous hearing implant of claim 1, **characterised in that** the adjusting pin (10) with the rotary holder (9.2) is made of UHMWPE polyethylene.

19. The intraosseous hearing implant of claim 1, **characterised in that** the adjusting pin (10) has a hole (10.01) together with a spherical groove (10.2), and a shank (9.2.1) of the holder, and a spherical tip (9.22) of the shank of the holder.

20. The intraosseous hearing implant of claim 1, **characterised in that** the fixing plate (11), which is approximately Y-shaped, has curved edges and a central surface (11.1) between the first arm (11.2) and the second arm (11.3) and the third arm (11.4).

21. The intraosseous hearing implant of claim 1, **characterised in that** the axis of the third arm (11.4) is at an angle of a = 120° with respect to the axis of the first arm (11.2) and the axis of the second arm (11.3).

22. The intraosseous hearing implant of claim 1, **characterised in that** the fixing plate (11) on the central surface (11.1) in its central part comprises a cylindrical socket (11.5) threaded internally over the entire surface.

23. The intraosseous hearing implant of claim 1, **characterised in that** the first arm (11.2) and the second arm (11.3) and the third arm (11.4) of the fixing plate (11) comprises working holes (11.6).

24. The intraosseous hearing implant of claim 1, **characterised in that** the fixing socket (11) is made of titanium alloy.

## Patentansprüche

1. Intraossäres Hörimplantat, das ein Teil eines auf Knochenleitung basierenden Systems zur Stimulierung der Hörwahrnehmung ist und aus einem mechanischen Schwingungserreger (1) besteht, wobei der Erreger einen elektromechanischen Wandler (2) sowie einen Schaft (3) und am gegenüberliegenden Ende ein kugelförmiges Endstück (4) aufweist, wobei der Schaft (3) des mechanischen Schwingungserregers (1) über eine Gleitkugel (8) verbunden ist, die eine Durchgangsbohrung (8.1) aufweist, durch die der Schaft (3) des mechanischen Schwingungserregers (1) verläuft, **dadurch gekennzeichnet, dass** das kugelförmige Endstück (4) mit einer Schutzhülle (5) aus Polymermaterial überzogen ist und eine Feststellmutter (6) aufweist, die am Endstück in Richtung des Schädelknochens (CZ) befestigt ist, sowie ferner einen Befestigungssitz (7), der betriebsmäßig tangential zur Feststellmutter (6) angeordnet ist, und dessen zum Schädelknochen (CZ) tangentiale Oberfläche mit einer Schicht aus bioaktiver Beschichtung überzogen ist und dessen Grundfläche bündig mit der Schutzhülle (5) des kugelförmigen Endstücks (4) des Schafts (3) des mechanischen Schwingungserregers (1) abschließt, und der mittels eines aus einem Biomaterial bestehenden Klebstoffs am Schädelknochen (CZ) befestigt ist, wobei der Schaft (3) des mechanischen Schwingungserregers (1) mit dem Einstellstift (10) verbunden ist, der in der Aufnahme (11.5) der Befestigungsplatte (11) sitzt, und zwar über eine feststehende Halterung (9.1) und eine drehbare Halterung (9.2), die beide an der Spitze des Einstellstifts (10) angeordnet sind, in der eine Gleitkugel (8) angeordnet ist.

2. Intraossäres Hörimplantat nach Anspruch 1, **dadurch gekennzeichnet, dass** der Befestigungssitz (7) eine zylindrische Form aufweist, deren tangential zur Oberfläche des Schädelknochens (CZ) verlaufende Grundfläche (A) flach ist.

3. Intraossäres Hörimplantat nach Anspruch 1, **dadurch gekennzeichnet, dass** der Befestigungssitz (7) eine mit einem Gewinde versehene äußere zylindrische Oberfläche aufweist.

4. Intraossäres Hörimplantat nach Anspruch 1, **dadurch gekennzeichnet, dass** der Befestigungssitz (7) aus Titan besteht.

5. Intraossäres Hörimplantat nach Anspruch 1, **dadurch gekennzeichnet, dass** der Klebstoff ein adhäsives, osteokonduktives und bioresorbierbares Biomaterial ist.

6. Intraossäres Hörimplantat nach Anspruch 1, **dadurch gekennzeichnet, dass** die tangential zur Oberfläche des Schädelknochens (CZ) verlaufende Grundfläche (A) des Befestigungssitzes (7) mit einer Hydroxylapatitschicht überzogen ist.

7. Intraossäres Hörimplantat nach Anspruch 1, **dadurch gekennzeichnet, dass** der Schaft (3) des mechanischen Schwingungserregers (1) eine zylindrische Form aufweist.

8. Intraossäres Hörimplantat nach Anspruch 1, **dadurch gekennzeichnet, dass** der Schaft (3) des mechanischen Schwingungserregers (1) aus Titan besteht.

9. Intraossäres Hörimplantat nach Anspruch 1, **dadurch gekennzeichnet, dass** die Schutzhülle (5) des kugelförmigen Endstücks (4) des Schafts (3) des mechanischen Schwingungserregers (1) aus Teflon besteht.

10. Intraossäres Hörimplantat nach Anspruch 1, **dadurch gekennzeichnet, dass** die Kammer des elektromechanischen Wandlers (2) die Form eines Zylinders mit einem Durchmesser d_{w} = 5 mm und einer Höhe 1_{w} = 6 mm aufweist.

11. Intraossäres Hörimplantat nach Anspruch 1, **dadurch gekennzeichnet, dass** die Feststellmutter (6) an der Schnittstelle zum Befestigungssitz eine zylindrische Form aufweist.

12. Intraossäres Hörimplantat nach Anspruch 1, **dadurch gekennzeichnet, dass** die Feststellmutter (6) im Kontaktbereich mit dem kugelförmigen Endstück (4) des Schafts (3) eine sphärische Vertiefung (7.1) aufweist.

13. Intraossäres Hörimplantat nach Anspruch 1, **dadurch gekennzeichnet, dass** der Einstellstift (10) eine zylindrische Form aufweist.

14. Intraossäres Hörimplantat nach Anspruch 1, **dadurch gekennzeichnet, dass** der Einstellstift (10) auf der mit der Befestigungsplatte (11) in Kontakt stehenden Oberfläche eine mit einem Gewinde versehene Außenfläche aufweist.

15. Intraossäres Hörimplantat nach Anspruch 1, **dadurch gekennzeichnet, dass** der Einstellstift (10) auf der Seite des Schafts (3) des mechanischen Schwingungserregers (1) eine feststehende Halterung (9.1) sowie eine drehbare Halterung (9.2) mit einer sphärischen Innenfläche aufweist.

16. Intraossäres Hörimplantat nach Anspruch 1, **dadurch gekennzeichnet, dass** die drehbare Halterung (9.2) in einem Drehwinkelbereich von bis zu 360° drehbar ist.

17. Intraossäres Hörimplantat nach Anspruch 1, **dadurch gekennzeichnet, dass** der Einstellstift (10) mit der feststehenden Halterung (9.1) aus einer Titanlegierung besteht.

18. Intraossäres Hörimplantat nach Anspruch 1, **dadurch gekennzeichnet, dass** der Einstellstift (10) mit der drehbaren Halterung (9.2) aus UHMWPE-Polyethylen besteht.

19. Intraossäres Hörimplantat nach Anspruch 1, **dadurch gekennzeichnet, dass** der Einstellstift (10) eine Bohrung (10.01) mit einer kugelförmigen Ausnehmung (10.2) und einen Schaft (9.2.1) der Halterung sowie ein kugelförmiges Endstück (9.22) des Schafts der Halterung aufweist.

20. Intraossäres Hörimplantat nach Anspruch 1, **dadurch gekennzeichnet, dass** die Befestigungsplatte (11), die eine annähernd Y-förmige Gestalt hat, abgerundete Kanten sowie eine zentrale Fläche (11.1) zwischen dem ersten Arm (11.2) und dem zweiten Arm (11.3) sowie dem dritten Arm (11.4) aufweist.

21. Intraossäres Hörimplantat nach Anspruch 1, **dadurch gekennzeichnet, dass** die Achse des dritten Arms (11.4) in einem Winkel a = 120° zur Achse des ersten Arms (11.2) und zur Achse des zweiten Arms (11.3) steht.

22. Intraossäres Hörimplantat nach Anspruch 1, **dadurch gekennzeichnet, dass** die Befestigungsplatte (11) auf der zentralen Fläche (11.1) in ihrem mittleren Bereich eine zylindrische Aufnahme (11.5) aufweist, die über ihre gesamte Fläche innen mit einem Gewinde versehen ist.

23. Intraossäres Hörimplantat nach Anspruch 1, **dadurch gekennzeichnet, dass** der erste Arm (11.2), der zweite Arm (11.3) und der dritte Arm (11.4) der Befestigungsplatte (11) Befestigungsöffnungen (11.6) aufweisen.

24. Intraossäres Hörimplantat nach Anspruch 1, **dadurch gekennzeichnet, dass** der Befestigungssitz (11) aus einer Titanlegierung besteht.

## Revendications

1. Implant auditif intra-osseux, faisant partie d'un système destiné à stimuler la perception auditive par conduction osseuse, qui se compose d'un générateur de vibrations mécaniques (1) comprenant un transducteur électromécanique (2) et comprenant une tige (3), et, à son extrémité opposée, un embout sphérique (4), la tige (3) du générateur de vibrations mécaniques (1) étant reliée au moyen d'une bille coulissante (8) comportant un trou traversant (8.1) à travers lequel passe la tige (3) du générateur de vibrations mécaniques (1), **caractérisé en ce que** l'embout sphérique (4) est recouvert d'un capuchon (5) en matière polymère et comporte un écrou de serrage (6) fixé sur l'embout en direction de l'os crânien (CZ), ainsi qu'un logement de fixation (7) qui est tangent à l'écrou de serrage (6), dont la surface en contact avec l'os crânien (CZ) est recouverte d'une couche de revêtement bioactif et dont la base affleure le capuchon (5) de l'embout sphérique (4) de la tige (3) du générateur de vibrations mécaniques (1) et qui est fixé à l'os crânien (CZ) à l'aide d'un adhésif constituant un biomatériau, la tige (3) du générateur de vibrations mécaniques (1) étant reliée à une tige de réglage (10) logée dans un logement (11.5) de la plaque de fixation (11) par l'intermédiaire d'un support fixe (9.1) et d'un support rotatif (9.2), tous deux situés au sommet de la tige de réglage (10), dans lequel est logée une bille de glissement (8).

2. Implant auditif intra-osseux selon la revendication 1, **caractérisé en ce que** le logement de fixation (7) a une forme cylindrique dont la surface de base (A) tangente à la surface de l'os crânien (CZ), est plane.

3. Implant auditif intra-osseux selon la revendication 1, **caractérisé en ce que** le logement de fixation (7) a une surface cylindrique extérieure filetée.

4. Implant auditif intra-osseux selon la revendication 1, **caractérisé en ce que** le logement de fixation (7) est réalisé en titane.

5. Implant auditif intra-osseux selon la revendication 1, **caractérisé en ce que** l'adhésif est un biomatériau adhésif, ostéoconducteur et biorésorbable.

6. Implant auditif intra-osseux selon la revendication 1, **caractérisé en ce que** la surface de la base (A) du logement de fixation (7) tangente à la surface de l'os crânien (CZ) est recouverte d'une couche d'hydroxyapatite.

7. Implant auditif intra-osseux selon la revendication 1, **caractérisé en ce que** la tige (3) du générateur de vibrations mécaniques (1) a une forme cylindrique.

8. Implant auditif intra-osseux selon la revendication 1, **caractérisé en ce que** la tige (3) du générateur de vibrations mécaniques (1) est réalisée en titane.

9. Implant auditif intra-osseux selon la revendication 1, **caractérisé en ce que** le capuchon (5) de l'embout sphérique (4) de la tige (3) du générateur de vibrations mécaniques (1) est réalisé en Téflon.

10. Implant auditif intra-osseux selon la revendication 1, **caractérisé en ce que** la chambre du transducteur électromécanique (2) a la forme d'un cylindre d'un diamètre d_{w} = 5 mm et d'une hauteur 1_{w} = 6 mm.

11. Implant auditif intra-osseux selon la revendication 1, **caractérisé en ce que** l'écrou de serrage (6) a une forme cylindrique au point de contact avec le logement de fixation.

12. Implant auditif intra-osseux selon la revendication 1, **caractérisé en ce que** l'écrou de serrage (6) présente une cavité sphérique (7.1) au niveau de la zone de contact avec l'embout sphérique (4) de la tige (3).

13. Implant auditif intra-osseux selon la revendication 1, **caractérisé en ce que** la tige de réglage (10) a une forme cylindrique.

14. Implant auditif intra-osseux selon la revendication 1, **caractérisé en ce que** la tige de réglage (10) présente une surface extérieure filetée sur la surface en contact avec la plaque de fixation (11).

15. Implant auditif intra-osseux selon la revendication 1, **caractérisé en ce que** la tige de réglage (10) comporte, du côté de la tige (3) du générateur de vibrations mécaniques (1), un support fixe (9.1) et un support rotatif (9.2) présentant une surface intérieure sphérique.

16. Implant auditif intra-osseux selon la revendication 1, **caractérisé en ce que** le support rotatif (9.2) peut tourner dans une plage allant jusqu'à 360° d'angle de rotation.

17. Implant auditif intra-osseux selon la revendication 1, **caractérisé en ce que** la tige de réglage (10) avec le support fixe (9.1) est réalisée en alliage de titane.

18. Implant auditif intra-osseux selon la revendication 1, **caractérisé en ce que** la tige de réglage (10) avec le support rotatif (9.2) est réalisée en polyéthylène UHMWPE.

19. Implant auditif intra-osseux selon la revendication 1, **caractérisé en ce que** la tige de réglage (10) comporte un trou (10.01) avec une rainure sphérique (10.2) ainsi qu'une tige (9.2.1) du support, et un embout sphérique (9.22) de la tige du support.

20. Implant auditif intra-osseux selon la revendication 1, **caractérisé en ce que** la plaque de fixation (11), qui a approximativement la forme de la lettre Y, comporte des bords arrondis et une surface centrale (11.1) située entre le premier bras (11.2), le deuxième bras (11.3) et le troisième bras (11.4).

21. Implant auditif intra-osseux selon la revendication 1, **caractérisé en ce que** l'axe du troisième bras (11.4) forme un angle a = 120° par rapport à l'axe du premier bras (11.2) et à l'axe du deuxième bras (11.3).

22. Implant auditif intra-osseux selon la revendication 1, **caractérisé en ce que** la plaque de fixation (11) comporte, sur sa surface centrale (11.1) dans sa partie médiane, un logement cylindrique (11.5) taraudé intérieurement sur toute sa surface.

23. Implant auditif intra-osseux selon la revendication 1, **caractérisé en ce que** le premier bras (11.2), le deuxième bras (11.3) et le troisième bras (11.4) de la plaque de fixation (11) comportent des ouvertures fonctionnelles (11.6).

24. Implant auditif intra-osseux selon la revendication 1, **caractérisé en ce que** le logement de fixation (11) est réalisé en alliage de titane.
